(19)

Europäisches
Patentamt

European
Patent Office

Office européen
des brevets

(11)　**EP 3 629 336 B1**

(12)　**EUROPEAN PATENT SPECIFICATION**

(45) Date of publication and mention
of the grant of the patent:
**26.03.2025 Bulletin 2025/13**

(21) Application number: **18197512.9**

(22) Date of filing: **28.09.2018**

(51) International Patent Classification (IPC):
**G16H 50/50** $^{(2018.01)}$　　**G16H 30/40** $^{(2018.01)}$
**G16H 40/63** $^{(2018.01)}$　　**A61C 7/00** $^{(2006.01)}$
**G06T 19/00** $^{(2011.01)}$　　**A61C 13/00** $^{(2006.01)}$

(52) Cooperative Patent Classification (CPC):
**G16H 30/40; A61C 7/002; A61C 13/0004;
G06T 19/006; G16H 40/63; G16H 50/50**

(54) **DENTAL DESIGN TRANSFER**

ÜBERTRAGUNG EINES ZAHNÄRZTLICHEN ENTWURFS

TRANSFERT DE CONCEPTION DENTAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**01.04.2020 Bulletin 2020/14**

(73) Proprietor: **Ivoclar Vivadent AG**
**9494 Schaan (LI)**

(72) Inventors:
• **Lancelle, Marcel**
**8049 Zürich (CH)**
• **Mörzinger, Roland**
**8050 Zürich (CH)**
• **Degen, Nicolas**
**8703 Erlenbach (CH)**
• **Sörös, Gabor**
**8041 Zürich (CH)**
• **Nemanja, Bartolovic**
**8046 Zürich (CH)**

(74) Representative: **Uexküll & Stolberg**
**Partnerschaft von**
**Patent- und Rechtsanwälten mbB**
**Beselerstraße 4**
**22607 Hamburg (DE)**

(56) References cited:
**US-A1- 2013 218 530**　　**US-A1- 2018 153 659**
**US-A1- 2018 168 781**

• **ALEKSANDR AMIRKHANOV ET AL: "WithTeeth:
Denture Preview in Augmented Reality", 1
January 2018 (2018-01-01), XP055574940, ISBN:
978-3-03868-072-7, Retrieved from the Internet
<URL:https://www.cg.tuwien.ac.at/research/
publications/2018/amirkhanov-2018-withteeth/
amirkhanov-2018-withteeth-paper.pdf>
[retrieved on 20190327], DOI: 10.2312/
VMV.20181250**
• **KAPANU AG: "Kapanu-Augmented Reality for
the Future of Dentistry.pdf", YOUTUBE, 6 March
2017 (2017-03-06), pages 1, XP054979194,
Retrieved from the Internet <URL:https://www.
youtube.com/watch?v=clEXAIN_QF4> [retrieved
on 20190307]**
• **SCHNABL D., W. PUELACHER: "Individuell
gefräster Kantsteg. -- Eine Langfristige
Investition", DENTALZEITUNG, vol. 2016, no. 2,
2016, pages 38 - 41, XP055935077, ISSN:
1615-2859**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**EP 3 629 336 B1**

**Description**

The present invention pertains to the field of dental augmented reality applications and more specifically to computer-implemented methods that allow to design a three-dimensional dental design model by using an augmented reality (AR) application, and to export the dental design model to another, e. g. external, application such as a computer-aided design (CAD) application..

For dental professionals and patients, it is of interest to get a visual impression of the appearance of the patient's face with a modified dental situation, i.e. to visualize the modified dental situation in an image of the face of the patient. Also, the appearance during or after a dental treatment may be of importance for the patient before deciding to undergo such treatment. For this purpose, a virtual preview (virtual mockup) of the dentition modified by dental treatment is helpful for the dentist and may also be used in the course of interactively modifying the treatment plan to get the most favourable aesthetic results.

US2018168781A1 discloses a method for displaying a three dimensional model on an AR display. A plurality of intraoral images of a patient from an intraoral scanner during an intraoral scanning procedure is received and a virtual three dimensional model of at least a portion of a dental arch is generated from the plurality of intraoral images. The three dimensional model, which will be displayed on the AR display, is based on intraoral scanned images of the dental situation of the patient. US2018168781A1 does not disclose model generation by an AR application, as well as following provision of a scan model and alignment of both models for export.

US2013218530A1 discloses a method of designing a dental restoration for a patient, wherein the method includes providing one or more 2D images, where at least one 2D image includes at least one facial feature; providing a 3D virtual model of at least part of the patient's oral cavity; arranging at least one of the one or more 2D images relative to the 3D virtual model in a virtual 3D space such that the 2D image and the 3D virtual model are aligned when viewed from a viewpoint, whereby the 3D virtual model and the 2D image are both visualized in the 3D space. US2013218530A1 does not disclose model generation by an AR application, as well as following provision of a scan model and alignment of both models for export.

US2018153659A1 discloses a method for planning, visualizing, and/or optimizing dental restoration on at least a part of pre-prepared teeth of a patient, based on at least one 3D digital model of at least a part of the pre-prepared teeth. This 3D digital model is then used for providing a 3D digital model of at least part of the prepared teeth, where the prepared teeth are provided by preparing the pre-prepared teeth by dental restorative work. Thus, in US2018153659A1 a 3D scan of the patient's dental situation is the basis for the created dental design model. US2018153659A1 does not disclose model generation by an AR application, as well as following provision of a scan model and alignment of both models for export.

The Youtube video of Kapanu AG with title "Kapanu-Augmented Reality for the Future of Dentistry" is a promotional video demonstrating the use of an augmented reality application in the field of dentistry and in particular the visualisation of a 3D dental model by an AR application based on a scan model. Specifically, the video shows images and videos of faces and mouth regions of people, wherein these images and videos are "augmented" in the sense that a visualization of a virtual object (here: virtual mock-up/dental design model) is superimposed in the mouth region. As a result, people can get a visual impression of the appearance of the persons face with a modified dental situation, i.e. with a denture corresponding to the dental design model. The video shows in different scenes various possible ways of designing a dental design model. Neither the export of the designed dental design model to another, e.g. external, application such as a computer-aided design application nor the technical method how to achieve such an export are shown in said video.

In the context of the present invention, the term "denture" is not necessarily restricted to full dentures but also comprises partial dentures, orthodontic situations or adaptations, or dental restorations such as dental prostheses, including crowns, crown lays, veneers, inlays and onlays, bridges, dental implants, and implant restorations. Accordingly, the term "dental model" includes all models of dental prostheses - such as models of complete and partial dentures - that are used for prosthodontic purposes.

It is desirable to create a denture design in an augmented reality application showing the design in the patient's face and then be able to easily export the design to another application such as a computer-aided design application, e. g. to create a denture, restoration or other prosthesis from the design.

It is therefore an object of the present invention to provide an improved method that allows transferring data of a three-dimensional dental design model from an augmented reality application to a computer-aided design application.

It is another object to provide such as method wherein a three-dimensional dental design model that has been designed in an augmented reality (AR) application is transferrable to a computer-aided design (CAD) or similar application.

It is a particular object to provide such a method wherein the three-dimensional dental design model transferred to the CAD or similar application can easily be used as a basis to plan a dental treatment.

It is a further object to provide such a method wherein a pose and/or scale of the three-dimensional dental design model is easily adaptable to fit the needs of the receiving CAD application.

It is another object to provide such a method that is performed fully or semi-automatically.

At least one of these objects is achieved by the method of claim 1 and/or one of the dependent claims of the present application.

The present invention pertains to a computer-implemented method for designing and exporting design data of a virtual three-dimensional dental design model from an augmented reality (AR) application, the AR application being adapted to visualize the dental design model with a preliminary pose and scale in an image of a face comprising a mouth region with a dental situation. The method comprises

- creating the dental design model using the augmented reality application, whereafter for exporting the dental design model the following steps are carried out:
- providing a three-dimensional scan model of the dental situation,
- aligning the scan model and the dental situation in the image to obtain a pose of the scan model, and
- calculating, based on the preliminary pose and scale and the pose of the scan model, at least one transformation to bring the design model and the scan model into a common coordinate system. The method further comprises obtaining an aligned design model by

   aligning the design model and the scan model in the common coordinate system, and

   calculating a corrected scale of the dental design model and applying the corrected scale so that the scale of the aligned design model matches the scale of the scan model. Calculating the corrected scale comprises calculating a distance of the dental design model to a virtual camera to match a distance of the scan model to the virtual camera.

In particular, the dental design model is brought to a coordinate system of the scan model.

An application to which the design data is exported can be a computer aided design (CAD) application or a similar software, for instance a 3D printer software. This other application can be an external application or an application that is connected to the AR application; for instance, the AR application and a CAD application can be incorporated as sub-applications into the same applications and/or be operable using the same graphical user interface.

According to one embodiment, the method comprises generating aligned design data based on the transformation, wherein the aligned design data comprises at least a surface geometry of the design model.

According to another embodiment, the method comprises generating aligned design data based on the transformation and obtaining an aligned design model by aligning the design model and the scan model in the common coordinate system, wherein the aligned design data comprises at least a surface geometry of the aligned design model.

According to another embodiment, the method comprises exporting the aligned design data to another application. In particular:

- exporting comprises generating an export file comprising the aligned design data;
- the aligned design data is exported in a file together with scan model data related to the scan model;
- the other application is an external application;
- the other application is a CAD application; and/or
- the export file is in STL file format.

According to another embodiment of the method, calculating the transformation comprises applying inverse transformation to the dental design model to maintain visually the same position of the dental design model with respect to the image.

According to another embodiment, the method comprises aligning the scan model to a known coordinate system before determining the transformation.

In one embodiment, aligning the scan model to the known coordinate system is performed automatically, using heuristics and/or statistical information.

In another embodiment, aligning the scan model to the known coordinate system comprises correcting a scale of the scan model, in particular by orders of magnitudes.

According to a further embodiment of the method, a model based alignment (e. g. edge based alignment) is used for aligning the dental situation of the scan model and the dental situation in the image, the model based alignment comprising at least:

- estimating a position of a camera used for capturing the image relative to the face in the image during recording of the image,
- rendering a two-dimensional image of the scan model using a virtual camera processing the scan model at the estimated position, and
- carrying out feature detection in the mouth region of the image recorded by the camera and in the rendered image by performing edge detection and/or a colour-based tooth likelihood determination in the respective images and forming a detected feature image for each detected feature.

A segmentation of teeth and gum in the scan model may be performed using colour information of the scan model and/or using heuristics with position and surface curvature information from the scan model, wherein determining the pose of the scan model is based on the segmentation. The invention also pertains to a computer programme product comprising programme code which is stored on a machine-readable medium, or being embodied by an electromagnetic wave comprising a programme code segment, and having computer-executable instructions for performing one of the above methods.

The invention in the following will be described in detail by referring to exemplary embodiments that are accompanied by figures, in which:

Fig. 1    shows the input and output of an exemplary method for exporting design data of a virtual 3D dental design model from an AR application to a CAD application;

Fig. 2    is a flow chart illustrating an exemplary embodiment of a method for exporting design data of a virtual 3D dental design model from an AR application to a CAD application;

Fig. 3    illustrates the step of aligning the 3D scan to a known coordinate system;

Figs. 4a-d    illustrate edge based alignment as an example for performing the step of improving the denture pose;

Figs. 5a-b    illustrate the step of segmenting teeth and gum in the 3D scan;

Figs. 6a-d    illustrate the step of correcting a scaling and distance of the 3D dental design model;

Figs. 7a-b    illustrate the step of exporting the 3D dental design model;

Fig. 8    shows the input and output of an exemplary method for visualizing a 3D dental design model in an AR application, which method is not covered by the present claims; and

Fig. 9    is a flow chart illustrating an exemplary embodiment of a method for visualizing a 3D dental design model in an AR application, which method is not covered by the present claims.

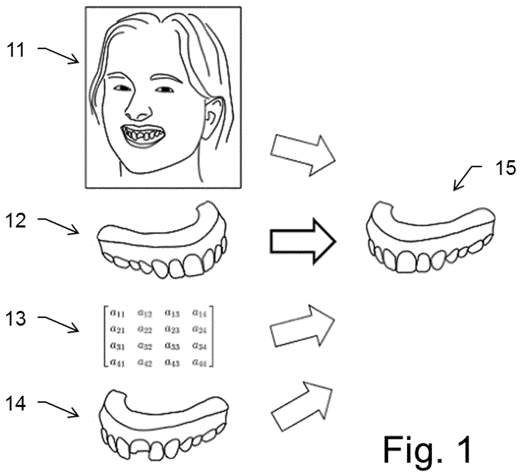

Figure 1 illustrates an exemplary embodiment of a method for exporting a 3D dental design model 12, bringing it into the coordinate system of a 3D scan of an actual dental situation. For instance, to plan a potential dental treatment, a preliminary dental design model is designed using an augmented reality application (AR app) and then transferred to a CAD software.

As illustrated in Figure **1,** the input comprises:

- at least one facial image 11 of a patient where at least a part of the mouth with the actual dental situation is visible,
- a 3D dental design model 12, e. g. generated by a user using said AR app,
- an estimated pose 13 of the 3D design model 12 to fit into the image 11, and
- a 3D scan model 14 of the oral cavity of the face depicted in the image 11 comprising 3D information about the actual dental situation.

The desired output is an aligned 3D dental design model 15, i.e. a three-dimensional model of designed teeth in the same coordinate system as the scan model 14 and matching the scale of the scan model 14.

The 3D scan model 14 may have been created by scanning the oral cavity of the patient or by scanning the impression of the dentition taken by impression trays filled with impression material. Creating the 3D scan model 14 may be part of the method. Alternatively, an existing model may be retrieved. Such three-dimensional model of the dentition of the patient may already be present, e. g. as a basis for developing a digital dental treatment plan, for example by adding artificial teeth or other dental restorations or by modifying the dental situation in another manner, for example by correction of teeth positions. The 3D scan model 14 may comprise the complete oral cavity or only parts thereof.

The 3D design model 12 can be a preliminary design. In particular, the 3D design 12 may have a wrong scale or be completely unscaled. The estimated pose 13 can be computed automatically with optional user adjustments in the AR app.

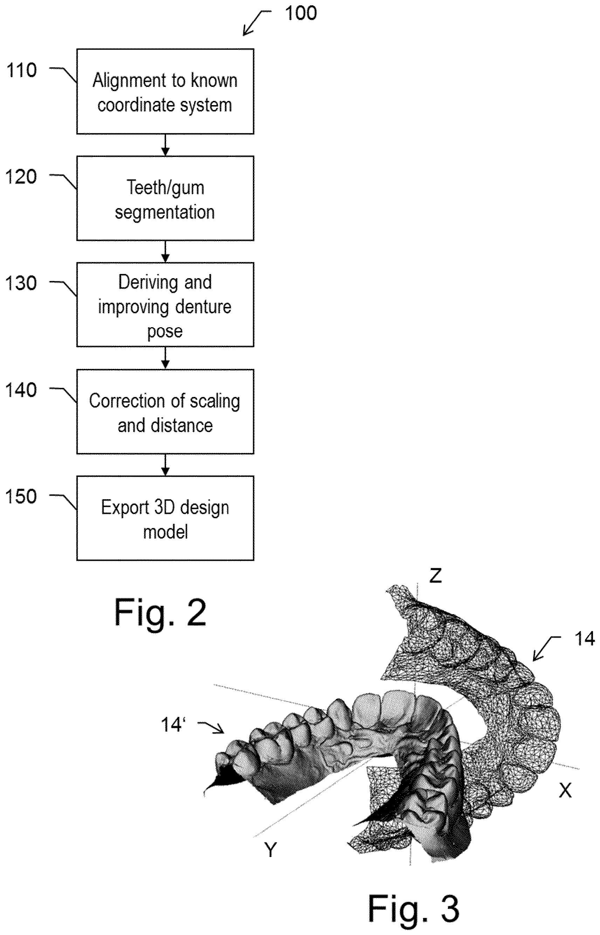

As illustrated by the flow chart of Figure 2, an exemplary embodiment of the method 100 comprises the following steps:

If the 3D scan model 14 is not already aligned (at least roughly) to the known coordinate system, this alignment is done in step 110. Figure 3 illustrates, by way of example, the original unaligned position of the 3D scan model 14 and the position of the aligned 3D scan model 14' after having performed said alignment 110. The alignment particularly comprises rotation and translation of the scan model 14. For instance, an automated system can use heuristics or statistical information to find the correct alignment, potentially also correcting the scale e. g. by orders of magnitudes. An auto-alignment, for instance, may comprise the following sub-steps:

1. Shifting a centroid of the 3D scan model 14 to the origin of the coordinate system (intersection of X, Y and Z axis);
2. applying a Principal Component Analysis (PCA);
3. snapping the PCA Y and X axes to the origin Y and X axes;
4. performing a series of flips:

a. Z-flip with condition that high curvature points are teeth and they have to point into $z > 0$ halfspace,
**b.** Z-flip with condition that open edges are on the gum endings and they have to point into $z < 0$ halfspace, and
c. Y-flip with condition that front teeth half-bounding box points into positive Y axis;

5. Performing translation adjustments including Y offset and occlusal plane.

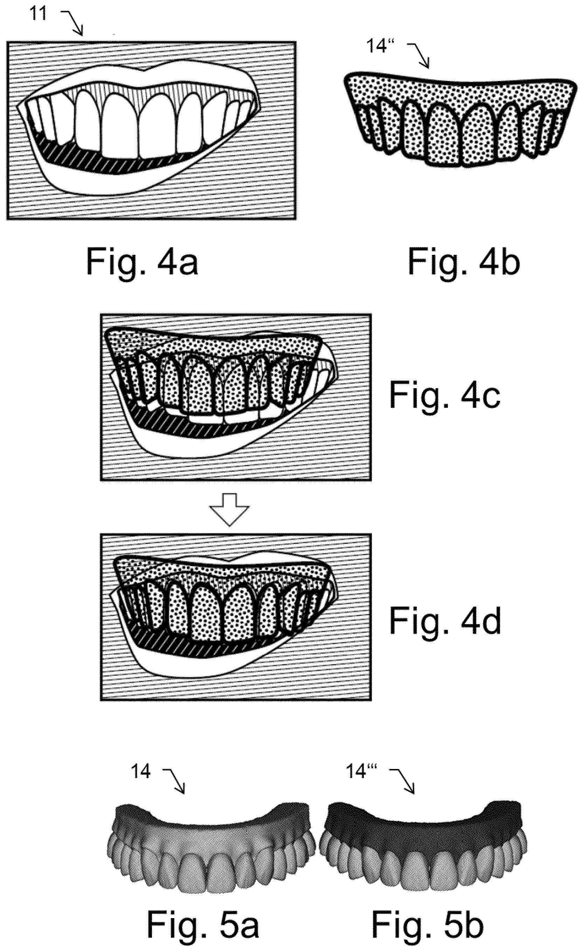

In step 130, the estimated denture pose 13 is further improved using edge based alignment. The edge based alignment is illustrated in Figures 4a-d and disclosed in detail in the applicant's European patent application 18157809.7. This improvement of the pose may be performed automatically with optional fine-tuning by a user.

It may comprise applying inverse transformation to design to keep the design visually at the same position with respect to the image.

The 3D scan model 14 of the denture is rendered by the virtual camera as a two-dimensional image of the denture, wherein the virtual camera is operated assuming an estimated positioning which is estimated to coincide with the positioning of the real camera when recording the image 11 of the patient's face. Preferably, an aperture angle of the camera optical system should to be known. If it is unknown, it can be estimated instead.

The image 11 of Figure 4a and the rendered image 14" of the 3D scan model of Figure 4b are then separately processed by carrying out feature detection in a dentition area inside the mouth opening in the respective images 11, 14" by performing edge detection and/or color-based tooth likelihood determination in the respective images 11, 14". The image 11 does not have to include the entire face, as the region of the mouth opening is sufficient.

For the detected feature (edges or tooth likelihood) or for each of the detected features (edges and tooth likelihood), this results in two detected feature images (one resulting from the camera image 11 and one from the rendered image 14") which are then used to calculate a measure of deviation between the detected feature images. Ideally, if the estimated positioning should already coincide with the real positioning of the camera when recording the face image, the measure of deviation would be zero or very small since the detected features (edges or tooth likelihood pattern) would be in identical positions in the two images 11, 14", and therefore there would be no deviation of the detected features in the two images 11, 14". However, in practice there will be a certain deviation at the beginning when an estimated position of the virtual camera is used. For this reason, the position of the virtual camera is varied to a new estimated position. This is iteratively repeated in an optimization process to minimize the deviation measure to determine the best fitting positioning of the virtual camera to arrive at the situation depicted in Figure 4d.

To facilitate the deriving of the pose, optionally, a rough segmentation 120 of teeth and gum can be performed before step 130. This can be done using heuristics with position and surface curvature information or (if available) using heuristics from colour information. Alternatively, if such a segmentation is already comprised in the mesh, that information can be used. This step is also illustrated in Figures 5a-b, Figure 5a showing a mesh without segmentation (teeth and gum having the same colour) and Figure 5b showing the same mesh after the segmentation 120 of teeth and gum (gum having a darker colour than teeth here).

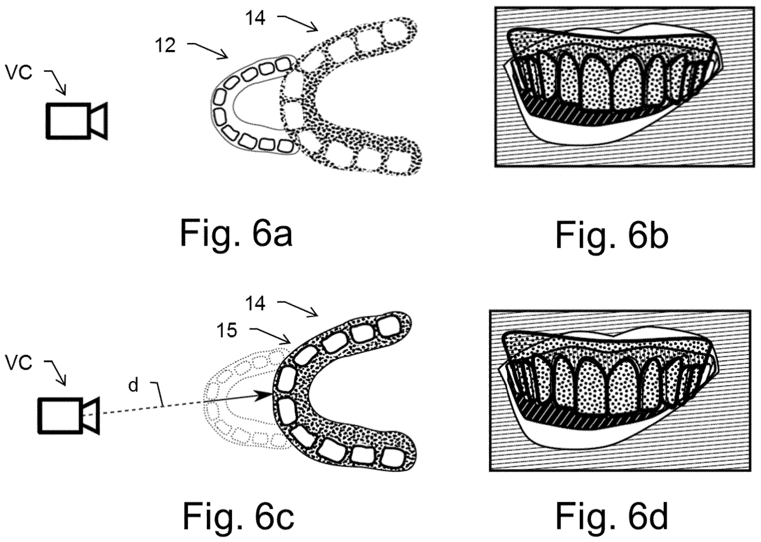

As illustrated in Figures 6a-d, the scaling and distance can be corrected 140 with the assumption that scan model 14 and dental design model 12 should be at a similar distance to a virtual camera VC. This step solves a potential problem that might occur when an unexperienced user designs the dental design model 12 in an augmented reality application and relates to an apparent size of the model in the AR application. Although the dental design model 12 may look correct, it could be too far in the front (and thus be smaller than intended) or too far behind the scan model 14 (and thus be larger than intended).

In Figure 6a, the dental design model 12 is much smaller than the scan model 14. As it is also positioned closer to the virtual camera VC, in the augmented reality image of Figure 6b, it appears to have the correct size. After having corrected the distance d to the dental design model 12 (see Figure 6c), the scan model 14 and the dental design model 12 can be aligned so that the aligned dental design model 15 has the correct scale, i.e. so that the apparent size in the image of Figure 6d is the same as in the image of Figure 6b.

Referring to Figure 2 again, finally, the aligned 3D design model 15 can be exported 150 (e. g. using STL file format) in the same coordinate system as the scan model 14. In particular, the model is exported to be used in a CAD application. However, the model can also be exported for use in other kinds of applications, such as, for instance, in a 3D printer.

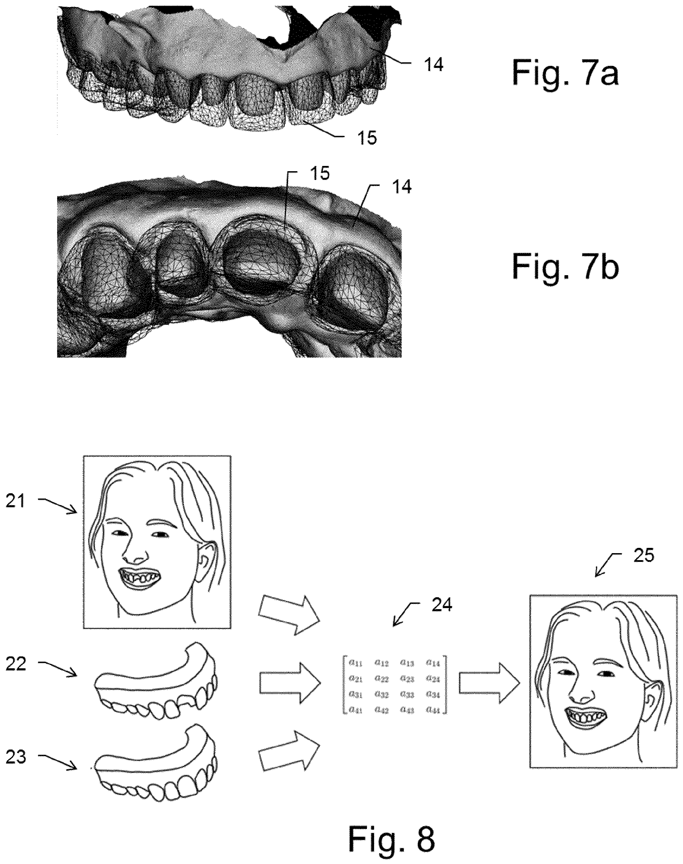

Advantageously, the 3D design model may be exported in the same file together with the scan model. Figures 7a-b illustrate the result by way of example, where the scan model 14 and the aligned 3D design model 15 are depicted aligned to each other.

In the following, an exemplary embodiment of the method 100 is described by way of equations. A 3D point of the design can be projected onto the image with

$$v_{design2D} = P_{cam} \times D_{app} \times M_{app} \times v_{design3D},$$

and the scan can be visualized in the image with:

$$v_{scan2D\_wrong} = P_{cam} \times D_{app} \times A \times v_{scan3D},$$

where:

- $P_{cam}$ is a 4×4 camera projection matrix that is computed from the image dimensions and the image field of view;
- D is a 4×4 denture pose, a transform that brings a point from denture coordinates to camera coordinates, $D_{app}$ being an estimated 4×4 denture pose from the AR app;
- M is a 4x4 design pose, $M_{app}$ being a transformation that the user specified with the user interface relative to $D_{app}$;
- $V_{design2D}$ is a point in the image in 2D;
- $v_{design3D}$ is a point on the model in 3D in the model coordinate system, i.e. the denture coordinate system;
- $v_{scan3D}$ is a vertex of the scan in 3D in an arbitrary coordinate system; and
- A is an alignment transform that brings the scan model (at least approximately) into the denture coordinate system (step 110).

However, the scan $v_{acan2D\_wrong}$ usually does not fit precisely, as the estimated denture pose $D_{app}$ and the alignment A both are not exact. An improved denture pose

$$D_{refined} = D_{app} \times D_{corr}$$

needs to be found, such that the fit is as good as possible:

$$v_{scan2D\_refined} = P_{cam} \times D_{refined} \times A \times v_{scan3D}.$$

$D_{corr}$ can be computed with edge based alignment or defined manually.

According to step 130, with the adjusted denture pose $D_{refined}$, the projection of the design should stay at the same position:

$$v_{design2D} = P_{cam} \times D_{app} \times D_{corr} \times D_{corr}^{-1} \times M_{app} \times v_{design3D}$$

$$v_{design2D} = P_{cam} \times D_{refined} \times M_{refined} \times v_{design3D}$$

with

$$M_{refined} = D_{corr}^{-1} \times M_{app}.$$

There is still an ambiguity in scale and distance from camera. As the true scale of the scan is known, the distance where it matches the image is known, too. Assuming that the distance d1 of the design from the camera should be the same as the distance d2 of the scan from the camera, the distance can be corrected with $T_{dist}$. For the visual result to stay the same, in step 140, the model needs to be scaled by d2/d1 with $T_{scale}$:

$$v_{design2D} = P_{cam} \times D_{refined} \times T_{dist} \times T_{scale} \times M_{refined} \times v_{design3D}.$$

Finally, in step 150, the design can be exported in the same coordinate system and same scale as the scan:

$$v_{design3Dexport} = A^{-1} \times T_{dist} \times T_{scale} \times M_{refined} \times v_{design3D}.$$

Figure 8 illustrates an exemplary embodiment of a method (not covered by the present claims) for visualizing an imported 3D dental design model 23 in an AR application, bringing a design resulting from a CAD or simulation (e. g. orthodontics) into a specific pose matching to a facial image 21 for an Augmented Reality overlay. The objective is to display the dental design in the image 21 in the mouth of a person. For instance, to preview a dental situation in the face of a patient, the situation is designed as a dental model using a CAD application and then transferred to an AR application software.

As illustrated in Figure 8, the input comprises at least one facial image 21 with at least part of the mouth visible, a 3D scan model 22 of the oral cavity, wherein the 3D scan model 22 has a coordinate system that is not necessarily aligned to the coordinate system of the face in the image 21, and the imported 3D dental design model 23, e. g. having been designed previously in a CAD application.

The output comprises a pose 24 of the 3D design model 23 corresponding to the image 21 and the corresponding rendered image 25.

The 3D scan 22 as well as the 3D dental design model 23 optionally may comprise colour information and/or a segmentation of teeth and gum.

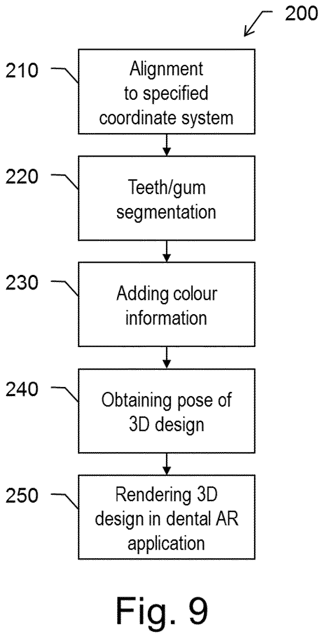

As illustrated by the flow chart of Figure 9, an exemplary embodiment of the method 200 (not covered by the present claims) comprises the following steps:

In a first step 210, the 3D scan 22 and the 3D design 23 are - at least roughly - aligned to a specified coordinate system. The same transformation is used for both 3D models 22, 23, i.e. both 3D models stay registered relative to each other. The 3D scan 22 and the 3D dental design model 23 optionally can be provided in the same coordinate system. Otherwise, they can be aligned to be in a common specified coordinate system.

A rough segmentation 220 of teeth and gum can then be performed in the 3D scan 22. This can be done using heuristics with position and surface curvature information or (if available) using heuristics from colour information. Alternatively, if such a segmentation is already comprised in the mesh of the 3D scan 22, that information can be used.

Especially if the 3D dental design model 23 does not already have colour information, in step 230 such colour information can be added to the model 23. This can be done either manually, semi- or fully automatically, e. g. using statistical information and heuristics. Colour information is important to let the design model 23 look more realistic in the AR application.

In step 240, the final pose 24 of the 3D design 23 is obtained. After step 210, the pose of the 3D design 23 is already in the specified coordinate system. The final pose 24 may be calculated using pose estimation from the dental AR application. The calculation for instance can be based on information about the segmentation of teeth and gum in the 3D scan 22 and on colour information from the 3D dental design model 23 (i.e. based on the information obtained in previous steps 220 and 230). The final pose 24 optionally can be further improved, e. g. by using a 3D scan for applying edge based alignment as described above with respect to Figures 4a-d and disclosed in the European patent application 18157809.7.

In the last step 250, the 3D dental design model 23 is rendered and a 2D representation of the model having the final pose 24 is displayed in the dental AR application, e. g. in the facial image 21 to provide the rendered image 25 as a result.

In an exemplary embodiment, a method for designing and exporting design data of a virtual three-dimensional dental design model is provided. The method includes providing an augmented reality visualizer configured to allow designing the dental design model by a user and displaying the dental design model. The design model has a preliminary pose and scale. The dental model is shown in an image of a face and the face image includes at least a portion of a mouth region and a dental situation. A 3D scan model of the dental situation is obtained. The scan model and the dental situation of the image are aligned to obtain a pose of the scan model. The method includes calculating, using a processor, at least one transform to bring the design model and the scan model into a common coordinate system based on the preliminary pose and scale and the pose of the scan model. The method further comprises obtaining an aligned design model by (i) aligning the design model and the scan model in the common coordinate system, and by (ii) calculating a corrected scale of the dental design model and applying the corrected scale so that the scale of the aligned design model matches the scale of the scan model. Calculating the corrected scale comprises calculating a distance of the dental design model to a virtual camera to match a distance of the scan model to the virtual camera.

An export dataset of aligned design data is generated based on the at least one transform. The aligned design data comprises at least one surface geometry of the design model. At least one model may be exported such that both models are aligned in a common coordinate system.

In the context of the present invention, the term "denture" is not necessarily restricted to full dentures but also comprises partial dentures or orthodontic situation/adaptations or dental restorations such as dental prostheses, including crowns, crown lays, veneers, inlays and onlays, bridges, dental implants, implant restorations. Accordingly, the term "dental model" includes all models of dental prostheses as well as the patient situation that could be partial or fully edentulous - such as models of complete and partial dentures - that are used for prosthodontic purposes.

In some embodiments, the present disclosure is implemented using a system having a camera, a processor, an electronic data storage unit, and a display. The camera can be a standard camera, an infrared dot-projection detector, flood illuminator camera, structured-light three-dimensional scanner, standard infrared detector, ultrasonic imaging device, Doppler detector, or any other suitable visualization system capable of capturing information related to a patient's dentition. The processor can be a single processor having one or more cores, or a plurality of processors connected by a bus, network, or other data link. The electronic data storage unit can be any form of non-transitory computer-readable storage medium suitable for storing the data produced by the system. The display can be any display suitable for displaying a digital color or grayscale image.

In some embodiments, the camera, processor, electronic data storage unit, and digital display are components of a single device. The single device may be a smartphone, tablet, laptop computer, personal digital assistant, or other computing device.

In some embodiments, the processor is in communication over a network, which could be wired or wireless, with an external processor used for performing one or more calculation steps and/or a network-attached electronic data storage unit. In some embodiments, the present disclosure makes use of cloud computing to perform one or more calculations steps remotely and/or remote storage to enable the storage of data remotely for collaborative or remote analysis. In some embodiments, the system comprises a plurality of graphical user interfaces to permit multiple users to view or analyze the same data.

In some embodiments, the system operates to provide one or more users with a visualization of a virtual dental model of a patient's teeth, which may be altered to visualize the effect of one or more dental or orthodontic alterations. In some embodiments, this allows the one or more users to visualize a "before" dentition image, i.e., the appearance of a patient's dentition prior to a dental or orthodontic procedure, and an "after" dentition image, i.e., a representation of the expected appearance of a patient's dentition after a proposed dental or orthodontic procedure.

In some embodiments, the system operates by capturing information related to a patient's dentition using a camera, creating a model of the patient's dentition on a processor, fitting a model of a proposed post-alteration dentition to the patient's dentition on the processor, coloring the model of the proposed post-alteration dentition to match an expected real post-alteration coloration, and displaying the fitted model of the proposed post-alteration dentition in place of the patient's actual dentition on a display which otherwise shows the patient's actual facial features. The information related to a patient's dentition, the model of the patient's dentition, and the model of the proposed post-alteration dentition may be stored on an electronic data storage unit. In some embodiments, the operations are performed in real-time.

In some embodiments, a user interface is configured such that a user may view the "before" dentition image and the "after" dentition image simultaneously either side-by-side or with a full or partial overlay.

Where used herein, the term "non-transitory" is a limitation on the computer-readable storage medium itself-that is, it is tangible and not a signal-as opposed to a limitation on the persistence of data storage. A non-transitory computer-readable storage medium does not necessarily store information permanently. Random access memory (which may be volatile, non-volatile, dynamic, static, etc.), read-only memory, flash memory, memory caches, or any other tangible, computer-readable storage medium, whether synchronous or asynchronous, embodies it.

## Claims

1.    Computer-implemented method (100) for designing a virtual three-dimensional dental design model (12) using an augmented reality application, the augmented reality application being adapted to visualize the dental design model (12) with a preliminary pose and scale (13) in an image (11) of a face comprising a mouth region with a dental situation, and for exporting the dental design model (12), the method comprising:

- creating the dental design model (12) using the augmented reality application, whereafter for exporting the dental design model the following steps are carried out:
- providing a three-dimensional scan model (14) of the dental situation,
- aligning the scan model (14) and the dental situation in the image (11) to obtain a pose of the scan model (14),
- calculating, based on the preliminary pose and scale (13) and the pose of the scan model (14), at least one transformation to bring the design model (12) and the scan model (14) into a common coordinate system, in particular wherein the dental design model (12) is brought to a coordinate system of the scan model (14), and
- obtaining an aligned design model (15) by

aligning the design model (12) and the scan model (14) in the common coordinate system, and calculating (140) a corrected scale of the dental design model (12) and applying the corrected scale so that the scale of the aligned design model (15) matches the scale of the scan model (14), wherein calculating (140) the corrected scale comprises calculating a distance of the dental design model (12) to a virtual camera (VC) to match a distance (d) of the scan model (14) to the virtual camera.

2. Method (100) according to claim 1,
**characterized by**
generating aligned design data based on the transformation, wherein

- the aligned design data comprises at least a surface geometry of the design model (12), or
- the method comprises obtaining an aligned design model (15) by aligning the design model (12) and the scan model (14) in the common coordinate system and the aligned design data comprises at least a surface geometry of the aligned design model (15).

3. Method (100) according to claim 2,
**characterized by**

exporting (150) the aligned design data to another application, particularly wherein

- exporting (150) comprises generating an export file comprising the aligned design data,
- the aligned design data is exported (150) in a file together with scan model data related to the scan model (14),
- the other application is an external application,
- the other application is a computer-aided design application, and/or

the export file is in STL file format.

4. Method (100) according to any one of the preceding claims,
**characterized in that**
calculating the transformation comprises applying inverse transformation to the dental design model (12) to maintain visually the same position of the dental design model (12) with respect to the image (11).

5. Method (100) according to any one of the preceding claims,
**characterized in that**
the method comprises aligning (110) the scan model (14) to a known coordinate system before calculating the transformation, particularly wherein aligning (110) the scan model (14) to the known coordinate system

- is performed automatically, using heuristics and/or statistical information, and/or
- comprises correcting a scale of the scan model (14), in particular by orders of magnitudes.

6. Method (100) according to any one of the preceding claims,
**characterized in that**
a model based alignment is used for aligning the dental situation of the scan model (14) and the dental situation in the image (11), the model based alignment comprising at least:

- estimating a position of a camera used for capturing the image (11) relative to the face in the image (11) during recording of the image,
- rendering a two-dimensional image (14") of the scan model using a virtual camera (VC) processing the scan model (14) at the estimated position, and
- carrying out feature detection in the mouth region of the image (11) recorded by the camera and in the rendered image (14") by performing edge detection and/or a colour-based tooth likelihood determination in the respective images (11, 14") and forming a detected feature image for each detected feature.

7. Method (100) according to any one of the preceding claims,
**characterized in that**
the dental design model (12) is designed or modified by a user using the augmented reality application, in particular wherein the method comprises designing or modifying the dental design model (12) using the augmented reality application.

8. Computer programme product comprising programme code which is stored on a machine-readable medium, or being embodied by an electromagnetic wave comprising a programme code segment, and having computer-executable instructions for performing the method according to any one of claims 1 to 7.

**Patentansprüche**

1. Computer-implementiertes Verfahren zum Entwerfen eines virtuellen, dreidimensionalen Dentalentwurfsmodells (12) unter Verwendung einer Augmented-Reality-Anwendung, wobei die Augmented-Reality-Anwendung dazu eingerichtet ist, das Dentalentwurfsmodell (12) mit einer vorläufigen Stellung und mit einer vorläufigen Größenskala (13) in einem Bild (11) eines Gesichts zu visualisieren, das eine Mundregion mit einer Dentalsituation enthält, und das Dentalentwurfsmodell (12) zu exportieren, wobei bei dem Verfahren:

   - das Dentalentwurfsmodell (12) unter Verwendung der Augmented-Reality-Anwendung erzeugt wird, wonach zum Exportieren des Dentalentwurfsmodells die folgenden Schritte ausgeführt werden:
   - Bereitstellen eines dreidimensionalen Abtastmodells (14) der Dentalsituation,
   - Ausrichten des Abtastmodells (14) und der Dentalsituation in dem Bild (11), um eine Stellung des Abtastmodells (14) zu erhalten,
   - Berechnen, basierend auf der vorläufigen Stellung und der vorläufigen Größenskala (13) und der Stellung des Abtastmodells (14), wenigstens einer Transformation, um das Entwurfsmodell (12) und das Abtastmodell (14) in ein gemeinsames Koordinatensystem zu bringen, wobei insbesondere das Dentalentwurfsmodell (12) in ein Koordinatensystem des Abtastmodells (14) gebracht wird, und
   - Erhalten eines ausgerichteten Entwurfsmodells (15) durch

      Ausrichten des Entwurfsmodells (12) und des Abtastmodells (14) in dem gemeinsamen Koordinatensystem und
      Berechnen (140) einer korrigierten Größenskala des Dentalentwurfsmodells (12) und Anwenden der korrigierten Größenskala, so dass die Größenskala des ausgerichteten Entwurfsmodells (15) zu der Größenskala des Abtastmodells (14) passt, wobei das Berechnen (140) der korrigierten Größenskala beinhaltet, einen Abstand des Dentalentwurfsmodells (12) zu einer virtuellen Kamera (VC) zu berechnen, um zu einem Abstand (d) des Abtastmodells (14) zu der virtuellen Kamera zu passen.

2. Verfahren (100) nach Anspruch 1, **gekennzeichnet durch**
   Erzeugen ausgerichteter Entwurfsdaten basierend auf der Transformation, wobei

   - die ausgerichteten Entwurfsdaten wenigstens eine Oberflächengeometrie des Entwurfsmodells (12) enthalten oder
   - das Verfahren beinhaltet, ein ausgerichtetes Entwurfsmodell (15) zu erhalten, indem das Entwurfsmodell (12) und das Abtastmodell (14) in dem gemeinsamen Koordinatensystem ausgerichtet werden, und die ausgerichteten Entwurfsdaten wenigstens eine Oberflächengeometrie des ausgerichteten Entwurfsmodells (15) enthalten.

3. Verfahren (100) nach Anspruch 2, **gekennzeichnet durch**
   Exportieren (50) der ausgerichteten Designdaten in eine andere Anwendung, wobei insbesondere

   - das Exportieren (150) beinhaltet, eine Exportdatei mit den ausgerichteten Entwurfsdaten zu erzeugen,
   - die ausgerichteten Entwurfsdaten in einer Datei zusammen mit den Abtastmodelldaten, die sich auf das Abtastmodell (14) beziehen, exportiert werden,
   - die andere Anwendung eine externe Anwendung ist,
   - die andere Anwendung eine Computer-Aided-Design-Anwendung ist, und/oder
   - die Exportdatei im STL-Dateiformat ist.

4. Verfahren (100) nach einem der vorhergehenden Ansprüche, **dadurch gekennzeichnet, dass**
   das Berechnen der Transformation beinhaltet, eine inverse Transformation auf das Dentalentwurfsmodell (12) anzuwenden, um visuell die gleiche Position des Dentalentwurfsmodells (12) in Bezug auf das Bild (11) beizubehalten.

**5.** Verfahren (100) nach einem der vorhergehenden Ansprüche, **dadurch gekennzeichnet, dass** das Verfahren beinhaltet, das Abtastmodell (14) zu einem bekannten Koordinatensystem auszurichten, bevor die Transformation berechnet wird, wobei insbesondere das Ausrichten (110) des Abtastmodells (14) zum bekannten Koordinatensystem

- automatisch durchgeführt wird unter Verwendung heuristischer und/oder statistischer Informationen, und/oder
- beinhaltet, eine Größenskala des Abtastmodells (14) zu korrigieren, insbesondere um Größenordnungen.

**6.** Verfahren (100) nach einem der vorhergehenden Ansprüche, **dadurch gekennzeichnet, dass** eine modellbasierte Ausrichtung zum Ausrichten der Dentalsituation des Abtastmodells (14) und der Dentalsituation im Bild (11) verwendet wird, wobei die modellbasierte Ausrichtung zumindest beinhaltet:

- eine Position einer zur Aufnahme des Bildes (11) verwendeten Kamera relativ zum Gesicht in dem Bild (11) während der Aufnahme des Bildes zu schätzen,
- ein zweidimensionales Bild (14") des Abtastmodells unter Verwendung einer virtuellen Kamera (VC), die das Abtastmodell (14) an der geschätzten Position verarbeitet, zu erzeugen,
- Merkmalerkennung in der Mundregion des von der Kamera aufgenommenen Bildes (11) und in dem erzeugten Bild (14") auszuführen, indem Kantenerkennung durchgeführt wird und/oder eine farbbasierte Zahnwahrscheinlichkeitserkennung in den jeweiligen Bildern (11, 14") durchgeführt wird und für jedes erkannte Merkmal ein Bild des erkannten Merkmals gebildet wird.

**7.** Verfahren (100) nach einem der vorhergehenden Ansprüche, **dadurch gekennzeichnet, dass** das Dentalentwurfsmodell (12) durch einen Benutzer unter Verwendung der Augmented-Reality-Anwendung entworfen oder modifiziert wird, wobei das Verfahren insbesondere beinhaltet, das Dentalentwurfsmodell (12) unter Verwendung der Augmented-Reality-Anwendung zu entwerfen oder zu modifizieren.

**8.** Computer-Programm-Produkt, das Programm-Code aufweist, der auf einem maschinenlesbaren Medium gespeichert ist oder durch eine elektromagnetische Welle verwirklicht ist, die ein Programm-Codesegment aufweist, und der computer-ausführbare Instruktionen zum Durchführen des Verfahrens nach einem der Ansprüche 1 bis 7 enthält.

## Revendications

**1.** Procédé informatique (100) pour dessiner un modèle de dessin dentaire tridimensionnel virtuel (12) en utilisant une application de réalité augmentée, l'application de réalité augmentée étant adaptée pour visualiser le modèle de dessin dentaire (12) avec une pose et échelle préliminaire (13) dans une image (11) d'un visage comprenant une région de bouche avec une situation dentaire, et pour exporter le modèle de dessin dentaire (12), le procédé comprenant les étapes suivantes:

- créer le modèle de dessin dentaire (12) en utilisant l'application de réalité augmentée, après quoi pour exporter le modèle de dessin dentaire, les étapes suivantes sont exécutées:
- fournir un modèle de balayage tridimensionnel (14) de la situation dentaire,
- aligner le modèle de balayage (14) et la situation dentaire dans l'image (11) pour obtenir une pose du modèle de balayage (14),
- calculer, en se basant sur la pose et échelle préliminaire (13) et sur la pose du modèle de balayage (14), au moins une transformation pour mettre le modèle de dessin (12) et le modèle de balayage (14) dans un système de coordonnées commun, en particulier dans lequel le modèle de dessin dentaire (12) est mis dans un système de coordonnées du modèle de balayage (14), et
- obtenir un modèle de dessin aligné (15) par les opérations suivantes:

aligner le modèle de dessin (12) et le modèle de balayage (14) dans le système de coordonnées commun, et calculer (140) une échelle corrigée du modèle de dessin dentaire (12) et appliquer l'échelle corrigée de telle manière que l'échelle du modèle de dessin aligné (15) correspond à l'échelle du modèle de balayage (14), dans lequel le calcul (140) de l'échelle corrigée comprend le fait de calculer une distance entre le modèle de dessin dentaire (12) et une caméra virtuelle (VC) pour qu'elle corresponde à une distance (d) séparant le modèle de balayage (14) de la caméra virtuelle.

**2.** Procédé (100) selon la revendication 1, **caractérisé par** le fait de générer des données de dessin aligné basées sur la transformation, dans lequel:

- les données de dessin aligné comprennent au moins une géométrie de surface du modèle de dessin (12), ou
- le procédé comprend le fait d'obtenir un modèle de dessin aligné (15) en alignant le modèle de dessin (12) et le modèle de balayage (14) dans le système de coordonnées commun et les données de dessin aligné comprennent au moins une géométrie de surface du modèle de dessin aligné (15).

3. Procédé (100) selon la revendication 2, **caractérisé par** le fait d'exporter (150) les données de dessin aligné vers une autre application, en particulier dans lequel:

- l'exportation (150) comprend le fait de générer un fichier d'exportation comprenant les données de dessin aligné,
- les données de dessin aligné sont exportées (150) dans un fichier avec les données de modèle de balayage se rapportant au modèle de balayage (14),
- l'autre application est une application extérieure,
- l'autre application est une application de conception assistée par ordinateur, et/ou

le fichier d'exportation est au format de fichier STL.

4. Procédé (100) selon l'une quelconque des revendications précédentes, **caractérisé en ce que** le calcul de la transformation comprend le fait d'appliquer une transformation inverse au modèle de dessin dentaire (12) pour maintenir visuellement la même position du modèle de dessin dentaire (12) par rapport à l'image (11).

5. Procédé (100) selon l'une quelconque des revendications précédentes, **caractérisé en ce que** le procédé comprend le fait d'aligner (110) le modèle de balayage (14) sur un système de coordonnées connu avant de calculer la transformation, en particulier dans lequel l'alignement (110) du modèle de balayage (14) sur le système de coordonnées connu:

- est réalisé automatiquement, en utilisant l'heuristique et/ou des informations statistiques, et/ou
- comprend le fait de corriger une échelle du modèle de balayage (14), en particulier par ordre d'amplitude.

6. Procédé (100) selon l'une quelconque des revendications précédentes, **caractérisé en ce que** l'on utilise un alignement basé sur un modèle pour aligner la situation dentaire du modèle de balayage (14) et la situation dentaire dans l'image (11), l'alignement basé sur un modèle comprenant au moins les étapes suivantes:

- estimer une position d'une caméra utilisée pour capturer l'image (11) par rapport au visage dans l'image (11) pendant l'enregistrement de l'image,
- rendre une image bidimensionnelle (14") du modèle de balayage en utilisant une caméra virtuelle (VC) traitant le modèle de balayage (14) à la position estimée, et
- réaliser une détection d'élément dans la région de la bouche de l'image (11) enregistrée par la caméra et dans l'image rendue (14") en réalisant une détection de bord et/ou une détermination de vraisemblance de dent basée sur la couleur dans les images respectives (11, 14") et former une image d'élément détecté pour chaque élément détecté.

7. Procédé (100) selon l'une quelconque des revendications précédentes, **caractérisé en ce que** le modèle de dessin dentaire (12) est conçu ou modifié par un utilisateur utilisant l'application de réalité augmentée, en particulier dans lequel le procédé comprend le fait de concevoir ou de modifier le modèle de dessin dentaire (12) en utilisant l'application de réalité augmentée.

8. Produit de programme informatique comprenant du code de programme qui est stocké sur un support lisible par une machine, ou qui est incorporé dans une onde électromagnétique comprenant un segment de code de programme, et comportant des instructions exécutables par un ordinateur pour réaliser le procédé de l'une quelconque des revendications 1 à 7.

Fig. 1

110 — Alignment to known coordinate system

120 — Teeth/gum segmentation

130 — Deriving and improving denture pose

140 — Correction of scaling and distance

150 — Export 3D design model

100

Fig. 2

Fig. 3

11

Fig. 4a

14"

Fig. 4b

Fig. 4c

Fig. 4d

14

14""

Fig. 5a

Fig. 5b

Fig. 6b

Fig. 6d

Fig. 6a

Fig. 6c

Fig. 7a

Fig. 7b

Fig. 8

200

| 210 | Alignment to specified coordinate system |
| --- | --- |

↓

| 220 | Teeth/gum segmentation |
| --- | --- |

↓

| 230 | Adding colour information |
| --- | --- |

↓

| 240 | Obtaining pose of 3D design |
| --- | --- |

↓

| 250 | Rendering 3D design in dental AR application |
| --- | --- |

## Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018168781 A1
- US 2013218530 A1
- US 2018153659 A1
- EP 18157809